# EUROPEAN PATENT SPECIFICATION

(11) **EP 1 233 930 B1**
(45) Date of publication and mention of the grant of the patent: **28.05.2008**
(21) Application number: 00977761.6
(22) Date of filing: 01.12.2000
(51) Int. Cl.: C01G 23/047, C09C 1/36, A61Q 17/04, C09K 11/69

(54) **A PARTICLE COMPRISING A HOST LATTICE AND A GUEST, ITS PREPARATION AND USE IN ULTRAVIOLET LIGHT SCREENING COMPOSITIONS**
EIN TEILCHEN BESTEHEND AUS EINEM WIRTSGITTER UND EINEM GAST, SEINE HERSTELLUNG UND VERWENDUNG IN ULTRAVIOLETTLICHT ABSORBIERENDEN ZUSAMMENSETZUNGEN
PARTICULE COMPRENANT UN RESEAU HOTE ET UN INVITE, SA PREPARATION ET SON UTILISATION DANS DES COMPOSITIONS DE PROTECTION CONTRE LES ULTRAVIOLETS

(30) Priority: 01.12.1999 GB 9928438
(43) Date of publication of application: 28.08.2002
(60) Divisional application: 07016268.0
(73) Proprietor: ISIS INNOVATION LIMITED, Summertown, Oxford OX2 7SG (GB)
(72) Inventor: KNOWLAND, John, Sebastian University of Oxford, Oxford OX2 7AX (GB); DOBSON, Peter, James University of Oxford, Oxford OX1 3PJ (GB); WAKEFIELD, Gareth Nanox Limited, Kidlington, Oxford OX5 1PF (GB)
(74) Representative: Benson, John Everett
(86) International application number: PCT/GB2000/004587
(87) International publication number: WO 2001/040114

(56) References cited:
- WO-A-99/60994
- DE-A- 2 545 243
- US-A- 3 981 737
- US-A- 5 451 252
- PATENT ABSTRACTS OF JAPAN vol. 2000, no. 01, 31 January 2000 (2000-01-31) & JP 11 279019 A (SHISEIDO CO LTD), 12 October 1999 (1999-10-12)

## Description

The present invention relates to UV screening compositions, methods for their preparation and their use. The invention in particular relates to, for example, compositions comprising particulate oxides, their preparation and their use as, for example, paints, plastics, coatings, pigments, dyes and compositions for topical application, in particular, for example, sunscreens.

The effects associated with exposure to sunlight are well known. For example, painted surfaces may become discoloured and exposure of skin to UVA and UVB light may result in, for example, sunburn, premature ageing and skin cancer.

Commercial sunscreens generally contain components which are able to reflect and/or absorb UV light. These components include, for example, inorganic oxides such as zinc oxide and titanium dioxide.

Titanium dioxide in sunscreens is generally formulated as "micronised" or "ultrafine" (20-50 nm) particles (so-called microreflectors) because they scatter light according to Rayleigh's Law, whereby the intensity of scattered light is inversely proportional to the fourth power of the wavelength. Consequently, they scatter UVB light (with a wavelength of from 290 to 320 nm) and UVA light (with a wavelength of from 320 to 400 nm) more than the longer, visible wavelengths, preventing sunburn whilst remaining invisible on the skin.

However, titanium dioxide also absorbs UV light efficiently, catalysing the formation of superoxide and hydroxyl radicals which may initiate oxidations. The crystalline forms of TiO₂, anatase and rutile, are semiconductors with band gap energies of about 3.23 and 3.06 eV respectively, corresponding to light of about 385 nm and 400 nm (1 eV corresponds to 8066 cm⁻¹).

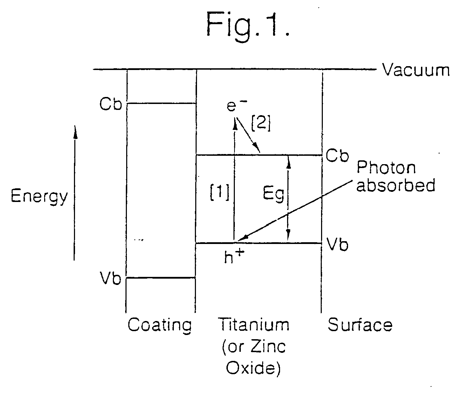

An incident photon is absorbed by titanium dioxide if its energy is greater than the semiconductor band gap Eg shown in Figure 1. As a result an electron from the valence band (Vb) is promoted into the conduction band (Cb) (transition [1]). If the energy of the incident photon is less than Eg it will not be absorbed as this would require that the electron be promoted to within the band gap and this energy state is forbidden. Once promoted, the electron relaxes to the bottom of the conduction band (transition [2]) with the excess energy being emitted as heat to the crystal lattice.

When the electron is promoted it leaves behind a hole which acts as a positive particle in the valence band. Both the electron and the hole are then free to migrate around the titanium dioxide particle. The electron and hole may recombine emitting a photon of energy equal to the band gap energy. However, the lifetime of the electron/hole pair is quite long due to the specific nature of the electronic band structure. Thus there is sufficient time (ca. 10⁻¹¹s) for the electron and hole to migrate to the surface and react with absorbed species.

In aqueous environments, the electrons react with oxygen, and the holes with hydroxyl ions or water, forming superoxide and hydroxyl radicals:

TiO₂ + hυ → TiO₂(e⁻/h⁺) → e⁻ (Cb) + h⁺ (Vb)

e⁻(Cb) + O₂ → O₂˙⁻ → HO˙₂

h⁺(Vb) + OH⁻ → ˙OH

This has been studied extensively in connection with total oxidation of environmental pollutants, especially with anatase, the more active form [A. Sclafani et al., J.Phys. Chem., (1996), 100, 13655-13661].

It has been proposed that such photo-oxidations may explain the ability of illuminated titanium dioxide to attack biological molecules. Sunscreen titanium dioxide particles are often coated with compounds such as alumina, silica and zirconia which form hydrated oxides which can capture hydroxyl radicals and may therefore reduce surface reactions. However, some TiO₂/Al₂O₃ and TiO₂/SiO₂ preparations exhibit enhanced activity [C. Anderson et al., J. Phys. Chem., (1997), 101, 2611-2616].

As titanium dioxide may enter human cells, the ability of illuminated titanium dioxide to cause DNA damage has also recently been a matter of investigation. It has been shown that particulate titanium dioxide as extracted from sunscreens and pure zinc oxide will, when exposed to illumination by a solar simulator, give rise to DNA damage both *in vitro* and in human cells [R. Dunford et al, FEBS Lett., (1997), 418, 87-90].
In our application No. PCT/WO99/60994 we describe and claim particles which comprise a host lattice incorporating a second component to provide luminescence trap sites and/or killer sites. The host lattice is typically TiO₂ and the second component is preferably manganese, but other metals namely nickel, iron, chromium, copper, tin, aluminium, lead, silver, zirconium, zinc, cobalt and gallium are also mentioned.

According to the present invention vanadium has been found to be effective. It may be incorporated singly or in combination with 2 or 3 or more of gallium, niobium, for example Nb⁵⁺, vanadium, for example V³⁺ or V⁵⁺, antimony for example Sb³⁺, tantalum, for example Ta⁵⁺, strontium, calcium, magnesium, barium, molybdenum, for example Mo³⁺, Mo⁵⁺ or Mo⁶⁺, silicon ions, Sn⁴⁺, Mn²⁺ Mn³⁺ and Co²⁺. Accordingly the present invention provides a UV screening composition which comprises a host lattice of zinc oxide or titanium dioxide incorporating a component to provide luminescence trap sites and/or killer sites, said second component being vanadium, said particles having an average primary particle size of from 1 to 100 nm, and a carrier. Preferred particles according to the present invention comprise a titanium dioxide host lattice doped with vanadium ions in the 5+ state.

It is believed that the presence of vanadium ions may make the host lattice more p-type. When a p-type particle absorbs light generating electrons and holes it is believed that any excess electrons are re-combined within the particle and so are prevented from leaving the particle and inducing reactions which may result in DNA damage.

It is also believed that with such particles the production of hydroxyl radicals is substantially reduced. Thus the production of hydroxyl radicals may be substantially prevented. The minimisation of migration to the surface of the particles of the electrons and/or the positively charged holes may be tested by, for example, looking for a reduction in the number of strand breaks inflicted on DNA by light in the presence of particles according to the present invention, as compared with the number of strand breaks observed in DNA on treatment with particles used in conventional sunscreen compositions and light, or light alone.

The average primary particle size of the particles is from 1 to 100 nm, preferably from 1 to 50 nm and more preferably from 20 to 50 nm. For example, in sunscreens the particle size is preferably chosen to avoid colouration of the final product. For this purpose particles of about 50 nm or less may be preferred especially, for example, particles of from 3 to 20 nm, preferably from 3 to 10 nm, more preferably from 3 to 5 nm.

Where particles are substantially spherical then particle size will be taken to represent the diameter. However, the invention also encompasses particles which are non-spherical and in such cases the particle size refers to the largest dimension.

The optimum amount of the second component in the host lattice may be determined by routine experimentation. It will be appreciated that the amount of the second component may depend on the use of the particles. For example, when the particles are used in UV screening compositions for topical application, it may be desirable for the amount of the second component in the host lattice to be low so that the particles are not coloured. Amounts as low as 0.1 % or less, for example 0.05%, or as high as 1% or above, for example 5% or 10%, can generally be used.

The dopant ions may be incorporated into the host lattice by a baking technique typically at a temperature of at least 300°C, generally at least 400°C and usually at least 600°C, for example 600°C to 1000°C, especially 650°C to 750°C eg. about 700°C. Thus, for example, these particles may be obtained in a known manner by combining particles of a host lattice with vanadium in the form of a salt such as a chloride or an oxygen-containing anion such as a perchlorate or a nitrate, in solution or suspension, typically in solution in water, and then baking it. A sufficient time should be allowed for the incorporation to be complete. Typically at least one hour is required, for example about 3 hours. Increasing the time further has generally little further effect.

Other routes which may be used to prepare the doped materials include a precipitation process of the type described in J. Mat. Sci. (1997) 36, 6001-6008. In this process solutions of the dopant salt and of an alkoxide of the host metal are mixed. The mixed solution is then heated to convert the alkoxide to the oxide. Heating is continued until a precipitate of the doped material is obtained. It will be appreciated that the precise temperatures of heating will depend on the nature of the alkoxide. Further, in the case of a titanium alkoxide, for example titanium isopropoxide, temperature will dictate whether the resulting titanium dioxide is in the anatase or rutile form. Generally, a higher temperature is used to obtain the anatase form.

It is believed that the dopant vanadium ions within the absorbing core act as localised sites and as such may exist within the band gap. Transitions [1] and [2] may occur as shown in Figure 1. However, the electron and hole may then relax to the excess V⁵⁺ sites. Thus the electrons and holes may be trapped so that they cannot migrate to the surface of the particles and react with absorbed species. The electrons and holes may then recombine at the V⁵⁺ sites accompanied by the release of a photon with an energy equivalent to the difference in the energy levels.

When the host is titanium dioxide it has been found that the presence of the second component enhances the conversion from anatase to rutile on baking; it appears that, surprisingly, the dopant ion has the effect of catalysing the conversion. It is believed that the dopant ion must be present in the lattice to achieve this result Thus on heating to at least, say, 530°C or 540°C, for example 600°C, at least 90% and generally at least 95%, for example 96 to 98%, of the anatase has been converted to the rutile form. The rutile form of titania is known to be more photostable than the anatase form. Heating undoped anatase to the same temperature results in significantly less conversion to rutile. Anatase starts to be converted to the rutile form at about 530°C to 540°C; although the extent of conversion increases as the temperature is raised, it remains only partial.

The particles of the present invention may have an inorganic or organic coating. For example, the particles may be coated with oxides of elements such as aluminium, zirconium or silicon. The particles of metal oxide may also be coated with one or more organic materials such as polyols, amines, alkanolamines, polymeric organic silicon compounds, for example, RSi[{OSi(Me)₂}xOR¹]₃ where R is C₁-C₁₀ alkyl, R¹ is methyl or ethyl and x is an integer of from 4 to 12, hydrophilic polymers such as polyacrylamide, polyacrylic acid, carboxymethyl cellulose and xanthan gum or surfactants such as, for example, TOPO.

The compositions of the invention may be used in a wide range of applications where UV screening is desired including paints, plastics, coatings and dyes, but are particularly preferred for topical application. The compositions for topical application may be, for example, cosmetic compositions including lipsticks, skin anti-ageing compositions in the form of, for example, creams, skin lightening compositions in the form of, for example, face powders and creams, compositions for protecting the hair and, preferably, sunscreens. Compositions of the present invention may be employed as any conventional formulation providing protection from UV light.

In effect the compositions of the present invention may be used to screen or protect a substrate from UV light as, for example, in sunscreens and/or screen or protect a UV sensitive component in the composition such as octyl methoxycinnamate, butyl methoxydibenzoyl methane or any of the following compounds:
(a) Para-aminobenzoic acids, esters and derivatives thereof, for example, 2-ethylhexyl para-dimethylaminobenzoate;
(b) methoxycinnamate esters such as 2-ethylhexyl para-methoxycinnamate, 2-ethoxyethyl para-methoxycinnamate or α,β-di-(para-methoxycinnamoyl)-α'-(2-ethylhexanoyl)-glycerin;
(c) benzophenones such as oxybenzone;
(d) dibenzoylmethanes such as 4-tert-butyl-4'methoxydibenzoylmethane;
(e) 2-phenylbeazimidazole-5 sulfonic acid and its salts;
(f) alkyl-β,β-diphenylacrylates for example askyl α-cyano-β,β-diphenylacrylates such as octocrylene;
(g) triazines such as 2,4,6-trianilino-(p-carbo-2-ethyl-hexyl-1-oxy)-1,3,5 triazine;
(h) camphor derivatives such as methylbenzylidene camphor.
(i) organic pigments sunscreening agents such as methylene bis-benzotriazole tetramethyl butylphenol;
(j) silicone based sunscreening agents such as dimethicodiethyl benzal malonate.

In compositions for topical application, the metal oxides are preferably present at a concentration of about 0.5 to 10 % by weight, preferably about 3 to 8 % by weight and more preferably about 5 to 7 % by weight. Such compositions may comprise one or more of the compositions of the present invention.

The compositions for topical application may be in the form of lotions, e.g. thickened lotions, gels, vesicular dispersions, creams, milks, powders, solid sticks, and may be optionally packaged as aerosols and provided in the form of foams or sprays.

The compositions may contain, for example, fatty substances, organic solvents, silicones, thickeners, demulcents, other UVA, UVB or broad-band sunscreen agents, antifoaming agents, moisturizing agents, perfumes, preservatives, surface-active agents, fillers, sequesterants, anionic, cationic, nonionic or amphoteric polymers or mixtures thereof, propellants, alkalizing or acidifying agents, colorants and metal oxide pigments with a particle size of from 100 nm to 20000 nm such as iron oxides.

The organic solvents may be selected from lower alcohols and polyols such as ethanol, isopropanol, propylene glycol, glycerin and sorbitol.

The fatty substances may consist of an oil or wax or mixture thereof, fatty acids, fatty acid esters, fatty alcohols, vaseline, paraffin, lanolin, hydrogenated lanolin or acetylated lanolin.

The oils may be selected from animal, vegetable, mineral or synthetic oils and especially hydrogenated palm oil, hydrogenated castor oil, vaseline oil, paraffin oil, Purcellin oil, silicone oil and isoparaffin.

The waxes may be selected from animal, fossil, vegetable, mineral or synthetic waxes. Such waxes include beeswax, Carnauba, Candelilla, sugar cane or Japan waxes, ozokerites, Montan wax, microcrystalline waxes, paraffins or silicone waxes and resins.

The fatty acid esters are, for example, isopropyl myristate, isopropyl adipate, isopropyl palmitate, octyl palmitate, C₁₂-C₁₅ fatty alcohol benzoates ("FINSOLV TN" from FINETEX), oxypropylenated myristic alcohol containing 3 moles of propylene oxide ("WITCONOL APM" from WITCO), capric and caprylic acid triglycerides ("MIGLYOL 812" from HULS).

The compositions may also contain thickeners which may be selected from cross-linked or non cross-linked acrylic acid polymers, and particularly polyacrylic acids which are cross-linked using a polyfunctional agent, such as the products sold under the name "CARBOPOL" by the company GOODRICH, cellulose, derivatives such as methylcellulose, hydroxymethylcellulose, hydroxypropyl methylcellulose, sodium salts of carboxymethyl cellulose, or mixtures of cetylstearyl alcohol and oxyethylenated cetylstearyl alcohol containing 33 moles of ethylene oxide.

When the compositions of the present invention are sunscreens they may be in a form of suspensions or dispersions in solvents or fatty substances or as emulsions such as creams or milks, in the form of ointments, gels, solid sticks or aerosol foams. The emulsions may further contain anionic, nonionic, cationic or amphoteric surface-active agents. They may also be provided in the form of vesicular dispersions of ionic or nonionic amphiphilic lipids prepared according to known processes.

Particularly when the particles are of titanium dioxide they are useful as pigments in paints. It is known that paints and varnishes undergo significant degradation in the presence of sunlight and/or UV light. The antioxidants currently used to counteract this are not wholly effective. The use of the inactivated titanium dioxide particles of the present invention significantly reduces the degradation of paints and varnishes and, in addition, contributes to a reduction in the "yellowing" of white formulations which occurs even in the dark but which is believed to be free , radical initiated. Paint formulations generally comprise pigments, binders or resins, solvents, and additives. The choice of binder or resin has a significant effect upon the performance properties of the paint. The preferred properties of the binder include the ability to cure under various conditions, good adhesion to various substrates, abrasive resistance, flexibility and water resistance. Typical binders include latex emulsions, alkyds, linseed oil, oil-modified epoxy and polyurethane resins and water-reducible alkyd and oil-systems. Generally the pigment volume concentration i.e. the total volume of pigment divided by the total volume of pigment and binder, expressed as a percentage, is from 15 to 75. The solvent is usually selected for its compatibility with the binder, and because it has the desired evaporation rate and toxicity profile. Typical solvents include mineral spirits, glycol solvents and other organic solvents. Additives are usually also included to either fulfil functions that are not covered by the other components or to assist the binder and the pigments to fulfil their particular functions. Typical additives include thickeners, driers, pigment dispersants, surfactants, defoamers and biocides.

### Brief Description of the Figures

Figure 1 shows the absorption of a photon of UV light by titanium dioxide as found in conventional sunscreens.
Figure 2 shows the effect on DNA of rutile TiO₂, undoped or doped with varying amounts of V⁵⁺ obtained by the precipitation process. The results were obtained by illumination of DNA in vitro as described in WO 99/60994.
Figure 3 shows the effect on DNA of V⁵⁺ doped TiO₂ obtained by the baking process.
Figure 4 shows the effect on DNA of TiO₂ doped with different dopants including the vanadium of the invention.
The Examples which follow further illustrate the present invention with reference to the figures.

### Example 1

### Preparation of vanadium doped titanium dioxide by baking

Titanium dioxide (25g) and ammonium vanadate (0.8g) were mixed in deionized water (100 ml). The resulting mixture was ultrasonicated for 10 minutes and then boiled dry. The material produced was fired at 700°C for 3 hours to give 1% vanadium doped titanium dioxide. Titanium dioxide particles with differing dopant levels were prepared in an analogous manner by varying the amount of ammonium vanadate.

### Example 2

### Preparation of doped rutile titanium dioxide by precipitation.

Water (720 ml; 40 moles) was mixed with concentrated hydrochloric acid (43 ml; 0.5 moles) and kept below 25 °C. Isopropanol (50 ml; 0.65 moles) was added slowly keeping the temperature below 25 °C.

For 1% doping, 0.0005 moles of dopant was added to the mixture and stirred until fully dissolved. Thus for ammonium metavanadate (MW = 116.98) 0.05849g was added.

Titanium isopropoxide (15 ml; 0.05 moles) was added dropwise with vigorous stirring with a magnetic stirrer until a translucent solution was produced. The solution was placed in a water bath at room temperature and slowly heated to 50°C and held at that temperature. After 1 to 4 hours the solution will begin to go cloudy as precipitation begins.

The temperature was held at 50°C until the precipitate had all settled and the solution had cleared (approximately 3 - 4 hours). The material was removed from the heat and allowed to settle for 12 hours.

As much supernatant as possible was pipetted off and the precipitate harvested by repeated centrifugation. The precipitate was washed twice with a mixture of water (44.3 ml), concentrated hydrochloric acid (2.6 ml) and isopropanol (3.1 ml). 15 ml of fluid was retained from the second wash and the wet precipitate was re-suspended and freeze dried.

### Example 3

### Preparation of doped anatase titanium dioxide by precipitation.

The procedure of Example 2 was repeated except for the heating step. In order to obtain the anatase form, the solution was heated rapidly (5°C/min) up to 90°C and held there until precipitation was complete (approximately 3½ hours).

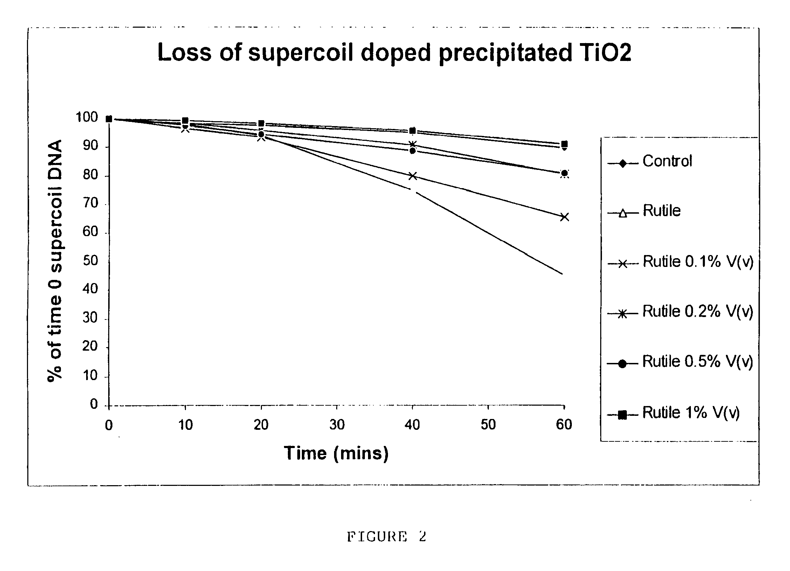

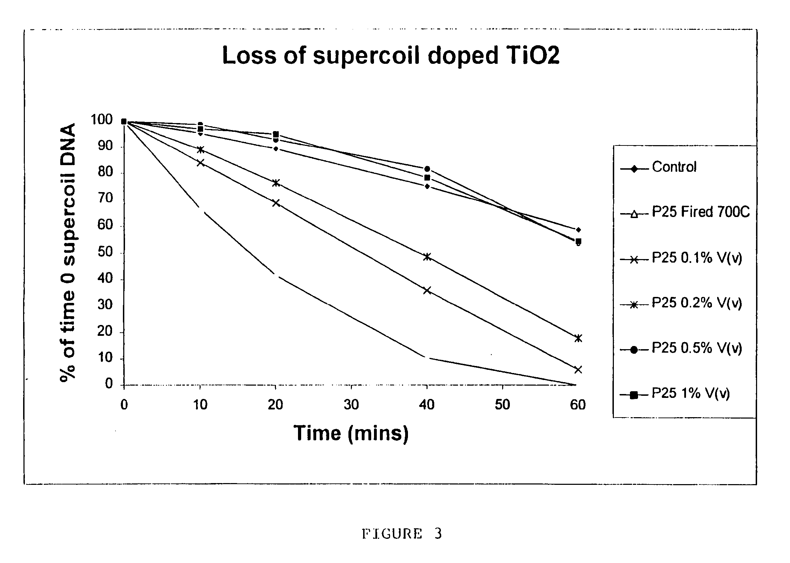

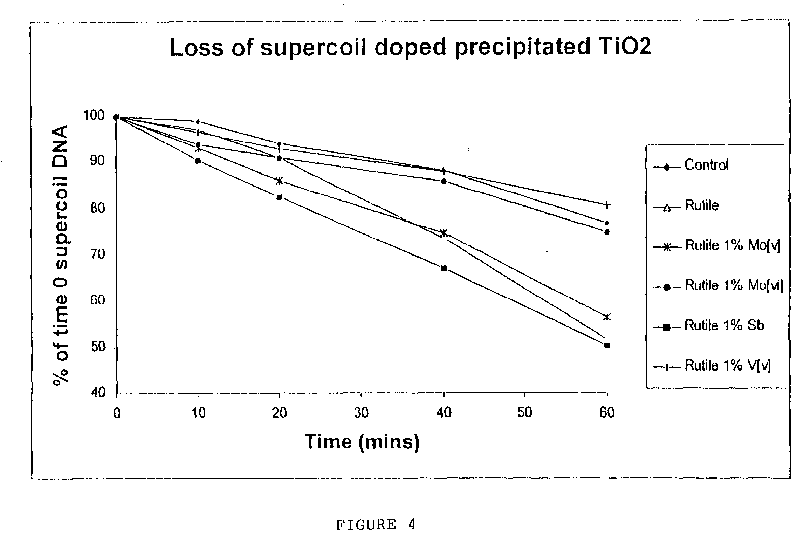

Figures 2, 3 and 4 show the effects obtained on DNA using these doped materials compared with undoped materials. P25 is a commercial grade of TiO₂.

### Example 4

### Preparation of oil-in-water sunscreen composition.

The following ingredients were used:

| Phase A | %ww |
|---|---|
| Glyceryl Stearate and PEG 100 Stearate | 5 |
| Sorbitan Stearate | 0.5 |
| Polysorbate 60 | 0.9 |
| Cetyl Alcohol | 1 |
| Liquid Paraffin | 8 |
| Sunflower oil | 5 |
| Dimethicone | 2 |

| Phase B | |
|---|---|
| Titanium Dioxide | 5 |
| Zinc Oxide | 2 |
| Xanthan Gum | 0.1 |
| Water | to 100 |

Phase A was heated to 70°C. To prepare Phase B, the Xanthan gum was dispersed into water and the resulting dispersion heated to 70°C. Using a high energy homogeniser the Titanium and Zinc were then dispersed into the hot Xanthan solution. Phase A was added to Phase B slowly and homogenised.

### Example 5

### Preparation of combined inorganic/organic sunscreen composition

The following ingredients were used:

| Phase A | %ww |
|---|---|
| Glyceryl Stearate and PEG 100 Stearate | 5 |
| Sorbitan Stearate | 0.5 |
| Polysorbate 60 | 0.9 |
| Cetyl Alcohol | 1 |
| Liquid Paraffin | 8 |
| Sunflower oil | 5 |
| Dimethicone | 2 |
| C12-15 Alcohols Benzoate | 5 |
| Octyl Methoxycinnamate | 4 |
| Butyl Methoxydibenzoyl Methane | 2 |
| | |

| Phase B | |
|---|---|
| Titanium Dioxide | 5 |
| Xanthan gum | 0.1 |
| Water | to 100 |

Phase A was heated to 70°C. To prepare Phase B, the xanthan gum was dispersed into water and the resulting dispersion heated to 70°C. Using a high energy homogeniser the Titanium Dioxide was then dispersed into the hot Xanthan solution.

Phase A was added to Phase B slowly and homogenised.

The following sunscreens are examples of organic filters that can be used in the above formulation to replace the methoxycinnamate and/or the dibenzoyl methane.
(a) Para-aminobenzoic acids, esters and derivatives thereof, for example, 2-ethylhexyl para-dimethylaminobenzoate;
(b) methoxycinnamate esters such as 2-ethylhexyl para-methoxycinnamate, 2-ethoxyethyl para-methoxycinnamate or α,β-di-(para-methoxycinnamoyl)-α'-(2-ethylhexanoyl)-glycerin;
(c) benzophenones such as oxybenzone;
(d) dibenzoylmethanes such as 4-tert-butyl-4'methoxydibenzoylmethane;
(e) 2-phenylbenzimidazole-5 sulfonic acid and its salts;
(f) alkyl-β,β-diphenylacrylates for example askyl α-cyano-β, β-diphenylacrylates such as octocrylene;
(g) triazines such as 2,4,6-trianilino-(p-carbo-2-ethyl-hexyl-1-oxy)-1,3,5 triazine;
(h) camphor derivatives such as methylbenzylidene camphor.
(i) organic pigments sunscreening agents such as methylene bis-benzotriazole tetramethyl butylphenol;
(j) silicone based sunscreening agents such as dimethicodiethyl benzal malonate.

### Example 6

### Preparation of water-in-oil sunscreen composition

| Phase A | %ww |
|---|---|
| Dimethicone and trimethylsiloxysilicate | 5 |
| Cyclomethicone | 8 |
| Laurylmethicone copolyol | 3 |
| Liquid Paraffin | 5 |
| | |

| Phase B | |
|---|---|
| Glycerine | 5 |
| Sodium Chloride | 1 |
| Titanium dioxide | 3 |
| Zinc Oxide | 8 |
| Water | to 100 |

The titanium dioxide and zinc oxide were dispersed into the other components of Phase B using a high energy homogeniser. Phases A and B were each heated to 70°C, then Phase A was slowly added to Phase B with stirring. The resulting mixture was homogenised to give the required viscosity.

## Claims

1. A UV screening composition which comprises particles which comprise a host lattice of zinc oxide or titanium dioxide incorporating a component to provide luminescence trap sites and/or killer sites, said component being vanadium, said particles having an average primary particle size of from 1 to 100 nm, and a carrier.

2. A composition according to claim 1 wherein the second component is vanadium in the 5+ state.

3. A composition according to claim 1 or 2 wherein the host lattice is titanium dioxide in rutile form.

4. A composition according to any one of claims 1 to 3 wherein the particles comprise a titanium dioxide host lattice doped with vanadium in the 5+ state.

5. A composition according to any one of the preceding claims wherein the particles are ones obtainable by baking titanium dioxide and a vanadium salt to a temperature of at least 600°C.

6. A composition according to any one of the preceding claims wherein the component is present in an amount of from about 0.1 to 1%.

7. A composition according to any one of the preceding claims which has a size is from 1 to 50 nm.

8. A composition according to any one of the preceding claims which has an outer coating.

9. A composition according to claim 8 wherein the outer coating is of an oxide of aluminium, zirconium or silicon.

10. A composition according to any one of the preceding claims which also comprises at least one UVA, UVB or broadband sunscreen agent.

11. A cosmetic product for topical application comprising a UV screening composition according to any of claims 1 to 10.

12. A cosmetic product according to claim 11 which is a sunscreen.

13. A product comprising a UV screening composition according to any one of claims 1 to 10, which product is a plastics composition, a pigment or a dye composition or a coating composition.

14. A composition according to claim 13 which is a paint.

15. Use of particles as defined in any one of claims 1 to 9, in a sunscreen composition for topical application, to screen UV radiation whilst minimising DNA damage to the host.

## Patentansprüche

1. UV-abschirmende Zusammensetzung, welche Partikel, die ein Wirtsgitter aus Zinkoxid oder Titandioxid umfassen, beinhaltend eine Komponente zur Bereitstellung von Lumineszenz-Einfangstellen und/oder -Killerstellen, welche Komponente Vanadium ist, wobei die Partikel eine mittlere primäre Partikelgröße von 1 bis 100 nm aufweisen, und einen Träger umfasst.

2. Zusammensetzung nach Anspruch 1, wobei die zweite Komponente Vanadium im 5+-Zustand ist.

3. Zusammensetzung nach Anspruch 1 oder 2, wobei das Wirtsgitter Titandioxid in Rutilform ist.

4. Zusammensetzung nach einem der Ansprüche 1 bis 3, wobei die Partikel ein Titandioxid-Wirtsgitter, das mit Vanadium im 5+-Zustand dotiert ist, umfassen.

5. Zusammensetzung nach einem der vorangehenden Ansprüche, wobei die Partikel solche sind, die durch Brennen von Titandioxid und einem Vanadiumsalz bei einer Temperatur von wenigstens 600 °C erhalten werden.

6. Zusammensetzung nach einem der vorangehenden Ansprüche, wobei die Komponente in einer Menge von etwa 0,1 bis 1 % vorhanden ist.

7. Zusammensetzung nach einem der vorangehenden Ansprüche, die eine Größe von 1 bis 50 nm aufweist.

8. Zusammensetzung nach einem der vorangehenden Ansprüche, die eine äußere Beschichtung aufweist.

9. Zusammensetzung nach Anspruch 8, wobei die äußere Beschichtung aus einem Oxid von Aluminium, Zirkonium oder Silizium ist.

10. Zusammensetzung nach einem der vorangehenden Ansprüche, die außerdem wenigstens ein UVA-, UVB- oder Breitband-Sonnenschutzmittel umfasst.

11. Kosmetisches Produkt für die topische Anwendung, umfassend eine UV-abschirmende Zusammensetzung nach einem der Ansprüche 1 bis 10.

12. Kosmetisches Produkt nach Anspruch 11, welches ein Sonnenschutz ist.

13. Produkt, umfassend eine UV-abschirmende Zusammensetzung nach einem der Ansprüche 1 bis 10, welches Produkt eine Kunststoffzusammensetzung, ein Pigment oder eine Farbstoffzusammensetzung oder eine Beschichtungszusammensetzung ist.

14. Zusammensetzung nach Anspruch 13, welche eine Anstrichfarbe ist.

15. Verwendung von Partikeln, wie in einem der Ansprüche 1 bis 9 definiert, in einer Sonnenschutz-Zusammensetzung für die topische Anwendung, um UV-Strahlung abzuschirmen und dabei eine DNA-Schädigung bei dem Wirt zu minimieren.

## Revendications

1. Composition de protection contre les UV qui comprend des particules contenant un réseau hôte d'oxyde de zinc ou de dioxyde de titane incorporant un composant pour fournir des sites de piégeage de luminescence et/ou des sites tueurs, ledit composant étant du vanadium, lesdites particules ayant une grosseur particulaire primaire moyenne de 1 à 100 nm, et un support.

2. Composition selon la revendication 1, dans laquelle le second composant est du vanadium à l'état d'oxydation +5.

3. Composition selon la revendication 1 ou 2, dans laquelle le réseau hôte est du dioxyde de titane sous sa forme rutile.

4. Composition selon l'une quelconque des revendications 1 à 3, dans laquelle les particules comprennent un réseau hôte de dioxyde de titane dopé au vanadium à l'état d'oxydation +5.

5. Composition selon l'une quelconque des revendications précédentes, dans laquelle les particules sont obtenues en cuisant au four du dioxyde de titane et du sel de vanadium à une température d'au moins 600 °C.

6. Composition selon l'une quelconque des revendications précédentes, dans laquelle le composant est présent dans une quantité d'environ 0,1 à 1 %.

7. Composition selon l'une quelconque des revendications précédentes qui possède une dimension de 1 à 50 nm.

8. Composition selon l'une quelconque des revendications précédentes qui possède un enrobage extérieur.

9. Composition selon la revendication 8, dans laquelle l'enrobage extérieur est un oxyde d'aluminium, de zirconium ou de silicium.

10. Composition selon l'une quelconque des revendications précédentes, qui comprend également au moins un agent écran solaire à large bande ou un agent protecteur contre les rayons UVA et UVB.

11. Produit cosmétique pour une application locale, comprenant une composition de protection contre les UV selon l'une quelconque des revendications 1 à 10.

12. Produit cosmétique selon la revendication 11 qui est un écran solaire.

13. Produit comprenant une composition de protection contre les UV selon l'une quelconque des revendications 1 à 10, lequel produit est une composition de matière plastique, une composition de pigment ou une composition de colorant ou une composition de revêtement.

14. Composition selon la revendication 13 qui est une peinture.

15. Utilisation de particules selon l'une quelconque des revendications 1 à 9, dans une composition d'écran solaire pour des applications locales, pour filtrer le rayonnement ultraviolet tout en minimisant les dommages à l'ADN de l'hôte.
